# EUROPEAN PATENT APPLICATION

(11) **EP 1 440 702 A1**
(43) Date of publication of application: **28.07.2004**
(21) Application number: 01974805.2
(22) Date of filing: 11.10.2001
(51) Int. Cl.: A61M 5/158

(54) **MEDICAL NEEDLE UNIT HAVING WING SHIELD FOR PREVENTING ERRONEOUS PUNCTURING**

(71) Applicant: JMS Co., Ltd., Hiroshima-shi, Hiroshima 730-8652 (JP)
(72) Inventor: KUNITOMI, Jun, Minato-ku, Tokyo 108-0072 (JP); DOI, Takashi, Hiroshima-shi, Hiroshima 731-0102 (JP)
(74) Representative: Schwarzensteiner, Marie-Luise, Dr.
(86) International application number: PCT/JP2001/008959
(87) International publication number: WO 2003/033055

(57) **Abstract**

A medical needle device includes a winged shield (4) having a substantially cylindrical shaped shield cylinder (4a) and wings (5, 6) connected to the front end side thereof, a needle base (2) inserted in the shield cylinder (4a), and a needle (1). The wings have wing projections (7, 8) and the shield cylinder has through holes (9, 10) on the wall thereof. By overlaying the wings along the side surface of the shield cylinder, each of the wing projections can be inserted into the inner cavity of the shield cylinder via the through hole. Thereby, without requiring a special operation in the sticking operation, each of the wing projections can prevent the needle base from moving in the axial direction and can hold the needle base in the shield cylinder reliably in a state in which the needle protrudes from the front end of the shield cylinder by a predetermined length.

## Description

### Technical Field

The present invention relates to a winged needle device for medical use. In particular, it relates to a medical needle device having a winged shield for preventing accidental needlesticks in which a needle can be stored safely after use.

### Background Art

Conventionally, in medical facilities, there have been problems of contamination or infection due to accidental needlesticks by an injection needle, a puncture needle, etc. Recently, in particular, since there has been a possibility that such an accidental needlestick might cause an infection of hepatitis B, hepatitis C, HIV (human immunodeficiency virus), or the like, increasing social attention is being paid to developments of means for positively preventing accidents such as an accidental needlestick.

As a means for preventing accidental needlesticks, various kinds of injection needle devices in which a needle is covered when an injection needle or a puncture needle is stored after use have been proposed. Most of such means for preventing accidental needlesticks have a cylindrical protection cover (which will be referred to as a shield, hereinafter) for preventing accidental needlesticks by a needle after use. The shield is slidable with respect to the injection needle. That is, by sliding the shield, the needle device alternately can be in a state in which the injection needle is exposed and a state in which the injection needle is covered with a shield.

On the other hand, for procedures such as an infusion of a medical solution, blood transfusion, extracoporeal blood circulation, or the like, winged injection needle devices are used widely. Such a winged injection needle device has a structure in which wings are attached to a needle base having a front end to which an injection needle is fixed and a rear end to which an infusion solution tube is connected. Therefore, the means for preventing accidental needlestick of the winged injection needle device requires a specific structure. That is, the means for preventing accidental needlestick must have a structure such that wings do not hinder the sliding of the shield. The structures of the conventional winged injection devices can be classified into a structure in which wings are attached to an injection needle or needle base and a structure in which wings are attached to a shield.

An example of the latter structure is disclosed in the official gazette of JP 6(1994)-7861B, WO91/04761, or the specification of US Patent No. 5,088,982. In these conventional examples, wings are attached to the outer surface of the slidable cylindrical shield and the wings slide outside the injection needle together with the shield. After use of the injection needle, it is possible to cover the tip of the injection needle by sliding the shield in order to prevent accidental needlesticks.

The above-mentioned injection needle device in which wings are attached to the shield needs a mechanism for temporarily holding an injection needle in a predetermined position with respect to a shield so that the injection needle is united with the shield. In particular, at the time of the sticking operation, the injection needle has to be held in the shield reliably. Unless this is the case, during the sticking operation, the injection needle may move in the shield, which seriously impacts the sticking operation. That is, there may be a possibility that the injection needle is pushed back to the base end side with the pressure at the time of the sticking, and thus the needle exposed from the shield is retracted.

Furthermore, while the needle is retained in the patient's body after a sticking operation, it is necessary to hold the injection needle in the predetermined position with respect to the shield. Furthermore, as mentioned below, it is desirable that the effect of holding the injection needle with respect to the shield is different between at the time of a sticking operation and at the time of a storing operation for storing the injection needle in the shield for preventing an accidental needlestick.

Since the sticking operation is carried out by grasping the shield, the needle is required to be held in the shield reliably. On the other hand, during the storing operation, the force for holding the needle with respect to the shield is preferably weak. If the holding force is too strong, the operation for sliding the needle inside the shield is difficult, which may lead to an unexpected accident. On the other hand, in a state in which the needle is retained in the patient's body, since the shield is fixed at the sticking site of the patient, if the needle easily moves inside the shield, there may be a possibility that the needle is pulled out from the patient's body. Therefore, when the needle is retained in the patient, a too weak force also is inconvenient. After all, it is desirable that after the sticking operation, a sufficiently weaker holding force as compared with the time of the sticking operation acts on the injection needle or the needle base.

However, the conventional injection needle device having a winged shield could not provide a reliable holding state of the injection needle with respect to the shield at the time of the sticking operation. That is, there may be a possibility that the holding is released due to its mechanism.

Furthermore, the holding of the injection needle is carried out by the common mechanism both during and after the sticking operation (when a needle is retained in the patient's body and when a needle is pulled out from the patient's body), and a suitable holding force cannot be obtained both during and after the sticking operation.

### DISCLOSURE OF THE INVENTION

It is an object of the present invention to provide a medical needle device capable of reliably holding an injection needle during a sticking operation.

Furthermore, it is an object of the present invention to provide a medical needle device capable of holding a medical needle with respect to a winged shield with an appropriate holding force and capable of carrying out the operation for storing a medical needle safely and easily during and after the sticking operation..

The medical needle device according to the present invention has a winged shield for preventing accidental needlesticks. The medical needle device includes a winged shield having a substantially cylindrical shaped shield cylinder and a pair of wings connected to the front end side of the shield cylinder, a needle base inserted into an inner cavity of the shield cylinder movably in the axial direction, and a needle attached to the front end of the needle base. The needle is capable of being stored in the inner cavity of the shield cylinder with the front end thereof being covered. Each of the wings has a wing projection protruding from the surface of the base region, and the shield cylinder has a through hole on the wall of the cylinder so that each of the wing projections can be inserted therein. By overlaying the wings along the side surface of the shield cylinder, each of the wing projections can be inserted into the inner cavity of the shield cylinder via the through hole. Thereby each of the wing projections prevent the needle base from moving in the axis direction inside the shield cylinder, and the needle base is held in the shield cylinder in a state in which the needle protrudes from the front end of the shield cylinder by a predetermined length.

With such a configuration, when the sticking operation is carried out, by grasping wings so that the portion of the wing projections are pinched to be pressed, it is possible to hold the needle base by the wing projections strongly and securely, thus enhancing the safety in sticking. Furthermore, since it is possible to enhance the force for holding the needle base at the time of sticking by only carrying out the same operation as that in a general winged needle without carrying out the special operation, the handling is easy.

In the above mentioned configuration, it is possible to employ a configuration in which each of the wing projections is inserted into the inner cavity of the shield cylinder via the through hole to be brought into contact with the needle base, and with the friction force caused by the contact, the needle base is held in the shield cylinder in a state in which the needle protrudes from the front end of the shield cylinder by a predetermined length.

Furthermore, it is preferable that the pair of through holes corresponding respectively to the pair of wing projections are formed separately on both the walls of the shield cylinder.

The through hole may be one groove formed continuously on both the side surfaces of the shield cylinder.

Furthermore, it is preferable that in each of the wings the thickness of the base region is smaller than the thickness of the edge side region in each of the wings.

Furthermore, it is preferable that the outer surface of the bottom portion of the shield cylinder is formed flat.

In the above-mentioned basic configuration, it is preferable that the needle base include a main tube portion and a holding portion located in the vicinity of the front end of the main tube portion, the holding portion has a large diameter portion whose diameter is larger than the diameter of the main tube portion, and a step portion is formed on the outer surface of the rear portion of the large diameter portion. By overlaying both the wings along the side surface of the shield cylinder, each of the wing projections can be engaged with the step portion of the needle base via the through hole. By engaging each of the wing projections with the step portion of the needle base, the needle base is held so that it does not move toward the base end side in the shield cylinder in a state in which the needle protrudes from the front end of the shield cylinder by a predetermined length.

With this configuration, the holding of the needle base becomes stable and strong.

In the above-mentioned basic configuration, it is preferable that the shield cylinder further includes a rear end latch portion formed on the inner surface of the rear end, and the rear end latch portion has a smaller inner diameter than that of the large diameter part in the holding portion of the needle base, and an annular groove is formed in the middle portion in the axial direction. By moving the needle base in the axial direction toward the base end side with respect to the shield cylinder, the large diameter portion in the holding portion of the needle base can be engaged with the annular groove in the rear end latch portion of the shield cylinder. The engagement enables the needle base to be held in the shield cylinder in a state in which the needle is stored in the inner cavity of the shield cylinder.

Furthermore, it is preferable that the holding portion of the needle base further includes an outward annular projection at the rear portion of the large diameter portion so as to form an annular groove on the outer surface between the large diameter portion and the outward annular projection. Each of the wing projections is inserted into the inner cavity of the shield cylinder via the through hole and can be engaged with the annular groove of the needle base. The engagement enables the needle base to be held in the shield cylinder in a state in which the needle protrudes from the front end of the shield cylinder by a predetermined length.

In this configuration, it is preferable that the shield cylinder further includes a rear end latch portion formed on the inner surface of the rear end. The rear end latch portion includes an inward annular projection; the inward annular projection can be engaged with the annular groove of the needle base by moving the needle base into the direction of the base end side with respect to the shield cylinder. The engagement enables the needle base to be held in the shield cylinder in a state in which the needle is stored in the inner cavity of the shield cylinder.

Preferably, the shield cylinder further includes a front end projection formed on the inner surface at a position in the axial direction substantially corresponding to the through hole. The front end projection of the shield cylinder can be engaged with the step portion of the needle base, and in the engagement state. By overlaying both the wings along the side surface of the shied cylinder, each of the projections can be engaged with the step portion of the needle base via the through hole. By engaging the front end projection or each of the wing projections with the step portion of the needle base, the needle base is held so that it is prevented from moving in the shield cylinder toward the base end side in a state in which the needle protrudes from the front end of the shield cylinder by a predetermined length.

With this configuration, during and after the sticking operation, the holding force for holding the medical needle with respect to the winged shield is suitably set, respectively.

In this configuration, it is preferable that the holding portion of the needle base further includes an outward annular projection at the rear portion of the large diameter portion so as to form the annular groove on the outer surface between the large diameter portion and the outward annular projection. By engaging the front end projection of the shield cylinder or each of the wing projections of the annular groove of the needle base, the same function as the holding function based on the step portion is seen.

Furthermore, it is preferable that by piercing and pressing the wings at the position of the each of the projections in a state in which both the wings are overlaid along the side surface of the shield cylinder and the step portion of the needle base is engaged with each of the wing projections, the holding force with respect to the needle base is capable of being made larger than the holding force obtained by the engagement between the step portion of the needle base and the front end projection.

In the above-mentioned basic configuration, it is preferable that a holding piece is formed on the upper surface of the shield cylinder.

Furthermore, preferably, the medical needle device according to claim 1, further including an auxiliary holding mechanism provided on the base end of the shield cylinder and an auxiliary holding portion formed on the rear portion of the needle base. The auxiliary holding mechanism includes a pivot piece attached to the outer surface of the base end portion of the shield cylinder, an auxiliary projection provided on the pivot piece, and a through hole provided on the wall of the shield cylinder so that the auxiliary projection can be inserted into the inner cavity of the shield cylinder by way of pivoting of the pivot piece, and the auxiliary holding portion includes an auxiliary annular groove or an auxiliary annular projection provided on the outer surface of the needle base. By engaging the auxiliary projection with the auxiliary annular groove or auxiliary annular projection of the needle base via the through hole, the auxiliary holding force for holding the needle base in the shield cylinder is provided in a state in which the needle protrudes from the front end of the shield cylinder by a predetermined length.

### BRIEF DESCRIPTION OF THE DRAWINGS

FIG. 1 is a sectional plan view showing of a medical winged needle device provided with a member for preventing an accidental needlestick according to a first embodiment of the present invention.
FIG. 2A is a sectional plan view showing a winged shield constituting the winged medical needle device of FIG. 1.
FIG. 2B is a sectional view taken on line A-A of FIG. 2A.
FIG. 3 is a sectional view taken on line B-B of FIG. 2A.
FIG. 4A is a front view showing a needle base constituting the medical needle device of FIG. 1.
FIG. 4B is a sectional view showing the needle base in the axial direction of FIG. 4A.
FIG. 5 is a sectional view showing an operation of the medical winged needle device of FIG. 1.
FIG. 6 is a side view showing a winged shield constituting a winged needle device according to a second embodiment of the present invention.
FIG. 7A is a plan view showing a winged shield constituting a medical winged needle device according to a third embodiment of the present invention.
FIG. 7B is a cross-sectional view taken on line D-D.
FIG. 8 is a front view showing a needle base constituting the medical winged needle device in the third embodiment of the present invention.
FIG. 9 is a sectional plan view showing a medical winged needle device according to a fourth embodiment of the present invention.
FIG. 10 is a sectional plan view showing a medical winged needle device according to a fifth embodiment of the present invention.

### BEST MODE OF CARRYING OUT THE INVENTION

### (First Embodiment)

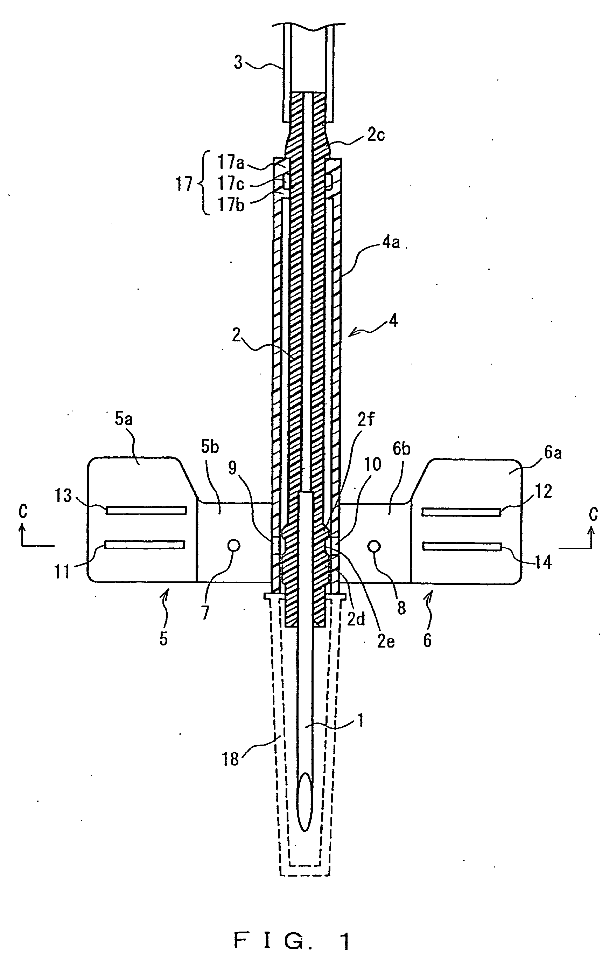

FIG. 1 is a plan view showing a medical needle device according to the first embodiment of the present invention. Reference numeral 1 denotes a needle that is fixed to the front end of a needle base 2 made of resin. To the rear end of the needle base 2, a tube 3 is connected. Reference numeral 4 denotes a winged shield, which includes a substantially cylindrical shaped shield cylinder 4a made of resin and left and right wings 5, 6. In the inner cavity of the shield cylinder 4a, the needle 1 and the needle base 2 are inserted movably in the axial direction. The left and right wings 5, 6 are provided at the front end of the shield cylinder 4a, that is, at the end from which the needle 1 protrudes. The wings 5, 6 are joined to both the side faces of the outer surface of the shield cylinder 4a, respectively. The wings 5, 6 are symmetrical with each other about the axis of the shield cylinder 4a as center. At the front end of the needle base 2, a needle cap 18 is fitted so as to cover the needle 1.

The wings 5, 6 include end regions 5a, 6a and base regions (regions at the side joined to the shield cylinder 4a) 5b, 6b. On the base regions 5b, 6b, the wing projections 7, 8 are formed, respectively. At the left and right walls of the shield cylinder 4a corresponding to the wing projections 7, 8, through holes 9, 10 are formed. On the end regions 5a and 6a, protruded stripe portions 11 and 12 and stripe grooves 13 and 14 are formed.

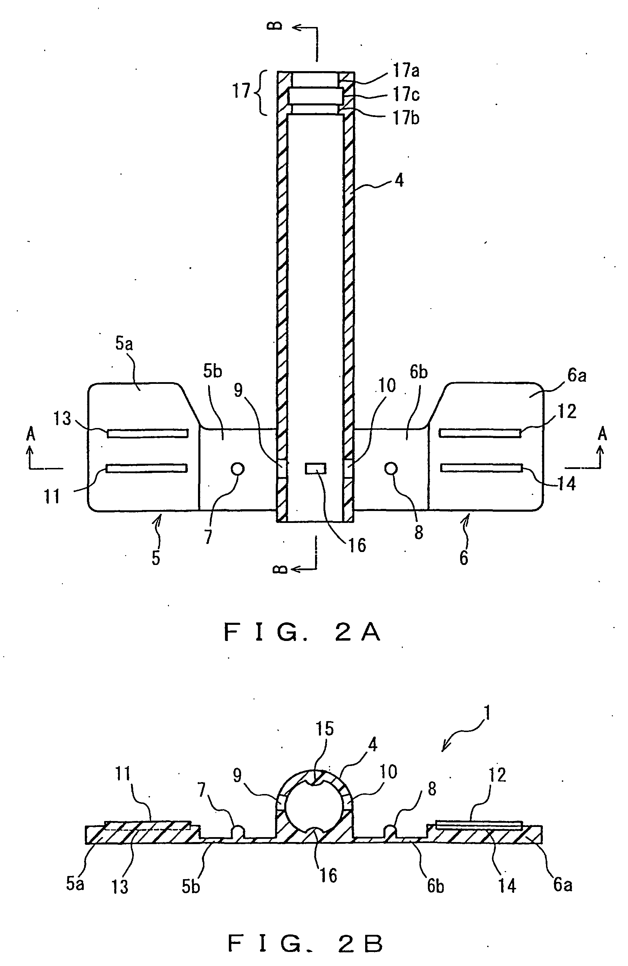

FIG. 2A shows a sectional shape of the winged shield 4. FIG. 2B shows a section taken on line A-A of FIG. 2A. The thickness of the base regions 5b, 6b is smaller than that of the end regions 5a, 6a in the wings 5, 6. Therefore, the wings 5, 6 can be bent easily at the base regions 5b, 6b.

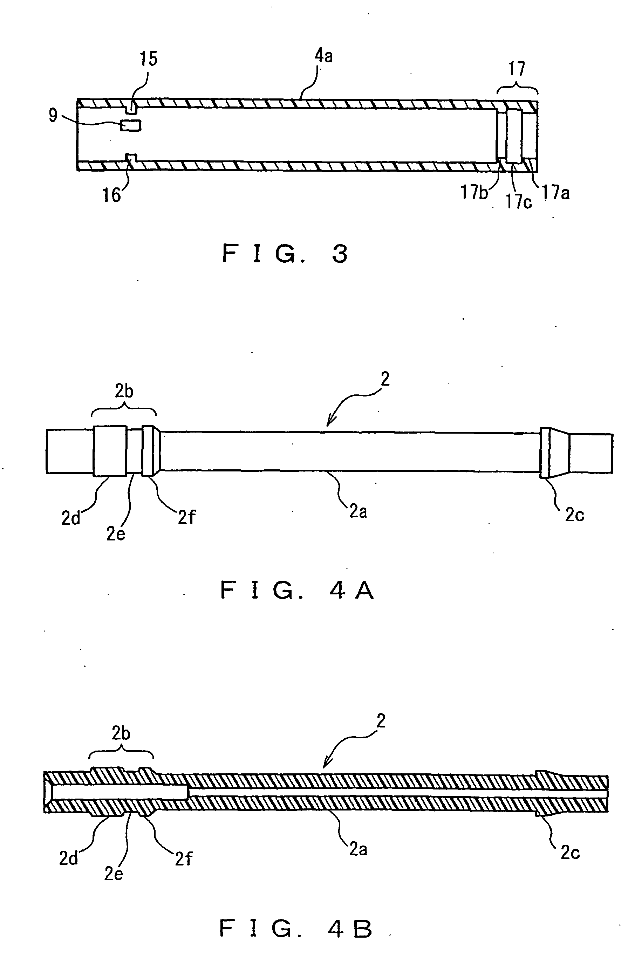

FIG. 3 shows a section in the axial direction of the shield cylinder 4a taken on line B-B of FIG. 2A. On the upper and lower surfaces of the inner surface of the front end of the shield cylinder 4a, the front end projections 15, 16 are formed. The positions in the axial direction of the front end projections 15, 16 substantially coincide with the positions of the through holes 9, 10. On the inner surface of the rear end of the shield cylinder 4a, a rear end latch portion 17 is formed. The rear end latch portions 17 includes a small diameter portion 17a and an inward annular projection 17b formed in the circumferential direction. The inward annular projection 17b is positioned to have a predetermined space from the small diameter portion 17a so as to thus form an annular groove 17c.

FIG. 4A shows an outer shape of the needle base 2, and FIG. 4B is a sectional view of the needle base 2 in the axial direction. The needle base 2 includes a main tube portion 2a at the central portion in the axial direction, a holding portion 2b formed at the front end, and a stopper portion 2c formed at the rear end. The outer diameter of the main tube portions 2a is smaller than the inner diameter of the small diameter portion 17a of the shield cylinder 4a and the inward annular projection 17b. Therefore, in the range in which the main tube portion 2a faces the small diameter portion 17a and the inward annular projection 17b, the shield cylinder 4a can be moved in the axial direction with respect to the needle base 2. The portion nearer to the front end than the holding portion 2b and the portion nearer to the rear end portion than the stopper portion 2c have the same diameter as that of the main tube portion 2a.

The outer diameter of the stopper portion 2c of the needle base 2 is larger than the inner diameter of the small-diameter portion 17a of the shield cylinder 4a. Therefore, when a step portion formed on the boundary between the stopper portion 2c and the main tube portion 2a is in contact with the small diameter portion 17a, that is, the rear end of the shield cylinder 4a, the needle base 2 is prevented from further moving toward the front end of the shield cylinder 4a. Thereby, the needle 1 is prevented from protruding from the shield cylinder 4a over a predetermined length.

The holding portion 2b of the needle base 2 includes a large diameter portion 2d, an annular groove 2e and an outward annular projection 2f. The outer diameters of the larger diameter portion 2d and the outward annular projection 2f are slightly larger than the space between the front end projections 15, 16 in the radial direction.

In an operation of attaching the winged shield 4 to the needle base 2, the needle base 2 is inserted from the front end of the shield cylinder 4a and moved toward the base end side. Firstly, the stopper portion 2c is in contact with the rear end latch portion 17. Since the rear end side of the stopper portion 2c has a taper shape, as shown in the drawing, it easily passes through the rear end latch portion 17 due to the flexibility of the resin. Substantially at the same time, the outward annular projection 2f of the needle base 2 is brought into contact with the front end projections 15, 16. Furthermore, by forcedly moving the needle base 2 toward the base end side, the front end projections 15, 16 pass beyond the outward annular projection 2f and are engaged with the annular groove 2e.

As a result, the state shown in FIG. 1 is obtained. The needle base 2 in FIG. 1 is in the position in use, and the needle 1 protrudes from the front end of the shield cylinder 4a. By the engagement between the front end projections 15, 16 and the annular groove 2e, the needle base 2 is prevented from moving in the shield cylinder 4a in the axial direction, so that the needle base 2 is held in the shield cylinder 4a. As mentioned above, in the state shown in FIG. 1, the needle 1 is prevented from further moving toward the front end side in the axial direction by the engagement between the stopper portion 2c and the small diameter portion 17a. Therefore, the main role of the engagement between the front end projections 15, 16 and the annular groove 2e is to hold the needle base 2 so that it does not move toward the base end side of the shield cylinder 4a.

Holding of the needle base 2 in the position for use provides a function to prevent an accident such that, for example, when the needle 1 is stuck and retained in the patient's body, the needle 1 moves toward the base end side in the winged shield 4 and drops off from the patient's body. On the other hand, when medical needle devices are disposed of after use, the needle 1 is stored in the shield cylinder 4a in order to avoid accidental needlesticks. At that time, since the needle base 2 is moved toward the rear end side of the shield cylinder 4a, too large holding force in the position for use makes the operation difficult. Therefore, the holding force in the position for use is set in the range in which the holding of the needle base 2 is not pulled out easily and the operation at the time of storing the needle 1 does not become difficult.

In this embodiment, the front end projections 15, 16 of the shield cylinder 4a, which are engaged with the annular groove 2e of the needle base 2, are formed partially only in the upper and lower parts of the inner surface. Thereby, the holding force by the engagement is adjusted to be relatively weak. However, the adjustment of the holding force is not necessarily limited to limiting the forming range of the front end projections 15, 16, and the holding force may be adjusted by changing the projecting length of the front end projections 15, 16 and other configurations, etc.

The outer diameters of the larger diameter portion 2d and the outward annular projection 2f are larger than the inner diameters of the small diameter portion 17a and the inward annular projection 17b of the shield cylinder 4a. Therefore, when the needle base 2 is moved in the direction toward the rear end of the shield cylinder 4a from the state shown in FIG. 1, thus releasing the engagement between the front end projections 15, 16 and the annular groove 2e and, furthermore the needle base 2 is moved in the direction toward the rear end, firstly, the outward annular projection 2f is in contact with the inner annular projection 17b. As shown in FIG. 4, since the outward annular projection 2f has a taper shape at the rear portion and the outer diameter of the outward annular projection 2f slightly is larger than the inner diameter of the inward annular projection 17b, by forcibly moving the needle base 2 further, the needle base 2 can pass through the inward annular projection 17b easily. When the outward annular projection 2f passes through the inward annular projection 17b, the outward annular projection 2f is engaged with the annular groove 17c. Furthermore, the inward annular projection 17b is engaged with the annular groove 2e. With such engagements, the needle base 2 is prevented from moving in the axial direction inside the shield cylinder 4a, resulting in a state in which the needle base 2 is held in the storing position in the shield cylinder 4a. In this storing position, the needle 1 is stored in the shield cylinder 4a, so that it is possible to obtain a state in which the accidental needlestick can be prevented.

The force for holding the needle base 2 in this storing position is required to be sufficiently large, because in general, it is not necessary to move the needle base 2 from the storing state for preventing the accidental needlestick toward the direction in which the needle 1 protrudes again and because it is desirable to hold the needle base 2 reliably in the storing position. The holding force for holding the needle base 2 at the storing position as in the above-mentioned configuration is sufficiently larger than the holding force at the position for use. Because, unlike the front end projections 15, 16, the engagement between the holding portion 2b of the needle base 2 and the rear end latch portion 17 of the shield cylinder 4a is made by the annular projections formed around the entire inner surface.

The rear end latch portion 17 may have a configuration in which the small diameter portion 17a is replaced by the inward annular projection 17b, that is, a configuration in which, at the rear end of the shield cylinder 4a, only the inward annular projection 17b is formed and the annular groove 17c is not formed. In such a case, the engagement between the holding portion 2b of the needle base 2 and the rear end latch portion 17 of the shield cylinder 4a is carried out only by the engagement between the inward annular projection 17b and the annular groove 2e.

FIGs. 5A to 5C show the operation of the winged projections 7, 8 provided on the wings 5, 6. These figures are sectional views taken on line C-C of FIG. 1, respectively. However, for easy understanding of the drawings, the hatching except for that of the needle base 2 is omitted in these drawings. Such an operation using the wings 5, 6 is carried out at the time of sticking. In this case, it is necessary that the needle base 2 is located in the position for use as shown in FIG. 1 and the annular groove 2e of the needle base 2 faces the through holes 9, 10. In general, since a medical needle device is used in a state shown in FIG. 1 and the needle base 1 is in an appropriate position at the time of sticking with respect to the winged shield 4, it is not necessary to carry out the positioning for use. Furthermore, according to this embodiment, since the state shown in FIG. 1 can be held by the engagement between the annular groove 2e and the front end projections 15, 16, displacement before sticking can be prevented.

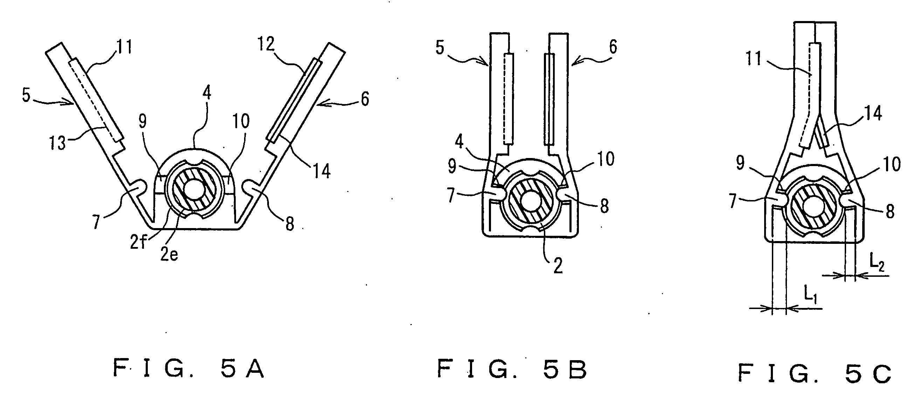

As shown in FIG. 5A, if the wings 5, 6 are lifted along the outer surface of the shield cylinder 4a, the wing projections 7, 8 face the through holes 9, 10. As shown in FIG. 5B, if the wings 5, 6 are lifted further, the wing projections 7, 8 are inserted into the through holes 9, 10, then penetrate the wall of the shield cylinder 4a, and protrude into the inner cavity. As a result, the tip ends of the wing projections 7, 8 are engaged with the annular groove 2e of the needle base 2. Thus, the needle base 2 with the needle 1 is held by the winged shield 4.

Furthermore, as shown in FIG. 5C, when the wings 5, 6 are put together, protruded stripe portions 11, 12 are fitted into stripe grooves 13, 14, respectively. Thus, both wings 5, 6 are put together with each other so that they are positioned in the predetermined relationship. Therefore, even if the wing protrusions 7, 8 are not appropriately inserted into the through holes 9, 10 at the stage as shown in FIG. 5B, mutual positional relationship can be corrected, thus reliably engaging them.

Note here that the device may be configured so that holding force at the time of sticking is obtained by friction without using the engagement between the annular groove 2e and the projections 7, 8. That is, in such a configuration, the tips of the projections 7, 8 are pressed onto the outer surface of the needle base 2 so as to prevent the needle base 2 from moving with the frictional force due to the pressing power.

In order to insert such wing projections 7, 8 smoothly, the mutual positional relationship between the wing projections 7, 8 and the through holes 9, 10 must be set precisely. Furthermore, as mentioned above, by reducing the thickness of the base end regions 5b, 6b, it is made easy to place the wings 5, 6 along the outer surface of the shield cylinder 4a when the wings 5, 6 are lifted upwardly.

The above-mentioned operation is carried out at the time of sticking, and the holding force exerted on the needle base 2 by the engagement between the wings 7, 8 and the needle base 2 is required to be sufficiently strong. That is, it is necessary to exert a larger holding force as compared with the holding force by the engagement between the front end projections 15, 16 and the annular groove 2e. The operation of sticking is carried out by pinching and pressing both wings 5, 6 with the finger, and a sufficient holding force can be obtained easily. However, it is necessary to set the shape and dimension of the wing projections 7, 8, appropriately. In particular, in the case where only the friction engagement between the needle base 2 and the wing projections 7, 8 is used without using the engagement with the annular groove 2e, the length of the wing projections 7, 8 has to be sufficiently large. For example, it is desirable that the length L1 of the wing projections 7, 8 is larger than the depth L2 of the through holes 9, 10. Specifically, it is desirable that L1 and L2 satisfy the relationship:
1 < L1/L2 ≤ 2.5.

The shape and dimension of the projections 7, 8 are determined so that they can easily be inserted into the through holes 9, 10. Furthermore, it is useful that the diameters of the projections 7, 8 are slightly smaller than the diameters of the through holes so that the wing projections 7, 8 are pulled out from the through holes 9, 10 immediately when the pressure is released.

In the above-mentioned example, the protruded stripe portion 11 and the stripe groove 13 are formed on the wing 5; and a protruded stripe portion 12 and a stripe groove 14 are formed on the wing 6, and the protruded stripe portion 11 and the stripe groove 14 and the protruded stripe portion 12 and the stripe groove 13 are engaged, respectively. The combination is not particularly limited to this. For example, one protruded stripe portion may be formed on one wing and the corresponding stripe groove may be formed on another wing. Furthermore, two protruded stripe portions may be formed on one wing and the corresponding two stripe grooves may be formed on another wing.

It is preferable that the shape of the inner cavity of the shield cylinder 4a corresponds to the shape of the outer surface of the needle base 2. For example, if the cross section perpendicular to the axis of the needle base 2 is a circular, it is preferable that the inner cavity of the shield cylinder 4a also is similarly circular or at least a part of the inner cavity is circular. In this embodiment, the inner cavity of the shield cylinder 4a has a circular shape, but it is possible to form the upper side of the inner cavity into a curved surface corresponding to the outer surface of the needle base 2 and form the bottom portion of the inner cavity surface flat. The bottom portion of the outer surface of the shield cylinder 4a is formed flat. In particular, the bottom portion of the outer surface of the shield cylinder 4a is preferably flat so that it can be placed stably on the skin of a patient.

Needless to say, the dimension of the shield cylinder is required to have such a length that the tip portion of the needle can perfectly be stored, other dimensions are not particularly limited. Preferably, the diameter of the inner cavity of the shield cylinder is slightly larger than the outer diameter of the needle base. For example, the configuration may be employed, in which the portion having the maximum outer diameter of the needle base is nearly in contact with the surface of the inner cavity of the shield cylinder, and the needle base and the needle move inside the inner cavity of the shield cylinder in the axial direction by sliding.

Next, the method for using the medical needle device in the above-mentioned embodiment will be explained. In use, as shown in FIG. 1, the medical needle device is provided in a state in which the winged shield 4 is attached to the needle base 2 to which the needle 1 is provided and the needle 1 is capped with the needle cap 18.

A user grasps the wings 5, 6 while putting them together with one hand and the needle cap 18 is removed from the needle 1 by another hand. Next, as shown in FIG. 5B or 5C, by lifting both the wings upwardly, the wings 5, 6 are grasped at the wing projections 7, 8. In this state, the wing projections 7, 8 penetrate the through holes 9, 10, and are engaged with the annular groove 2e of the shield cylinder 2e. Therefore, the needle 1 and the needle base 2 are held in the shield cylinder 4a in a way in which it cannot move in the shield cylinder 4a. In this state, the needle 1 is stuck into the patient's body.

When the needle 1 is stuck into a patient, both wings 5, 6, which are put together, are opened and then the wings 5, 6 are fixed onto the skin of the patient with adhesive tape. In this state, the projections 7, 8 are not engaged with the needle base 2 but the front end projections 15, 16 of the shield cylinder 4a are engaged with the annular groove 2e of the needle base 2. At this time, although the holding force is weaker than that in the case where the wings 5, 6 are used, the needle base 2 is held in the shield cylinder 4a. Therefore, it is possible to prevent the needle 1 from moving toward the base end side in the axial direction inside the shield cylinder 4a and to prevent the needle 1 from being pulled out from the patient's body.

When the infusion of drug solution into the patient's body is finished, the tube 3 is pulled toward the base end side of the shield cylinder 4a with the wings 5, 6 fixed or while pushing the wings 5, 6 by the hand. Thus, the engagement between the front end projections 15, 16 and the annular groove 2e with weak holding force is released, thus enabling the needle base 2 to move. Furthermore, if the tube 2 is pulled toward the base end side, the needle 2 including the tip is stored in the shield cylinder 4a perfectly and a state in which the accidental needlestick is prevented can be obtained. At the same time, the holding portion 2b of the needle base 2 and the rear end latch portion 17 of the shield cylinder 4a are engaged with each other, and thus are in the state in which the needle base 2 is strongly held in the storing position of the shield cylinder 4a.

### (Second Embodiment)

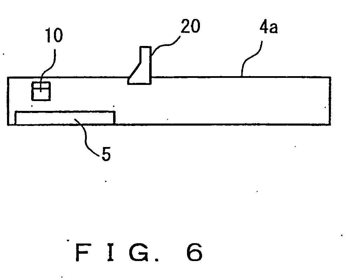

FIG. 6 shows a winged shield constituting a medical needle device according to a second embodiment. This winged shield includes a holding piece 20 on the upper surface of the shield cylinder 4a in addition to the configuration according to the embodiment 1. As mentioned above, after the medical needle device is used, by pulling the tube connected to the needle base toward the base end side of the shield cylinder 4a the needle base is held in the shield cylinder 4a in a state in which the accidental needlestick is prevented. The holding piece 20 is used for fixing the shield cylinder 4a so as not to move at this time. That is, by putting the holding piece 20 by the finger to resist the force of pulling the tube, the operation of pulling the needle base toward the base end side of the shield cylinder 4a becomes easy.

### (Third Embodiment)

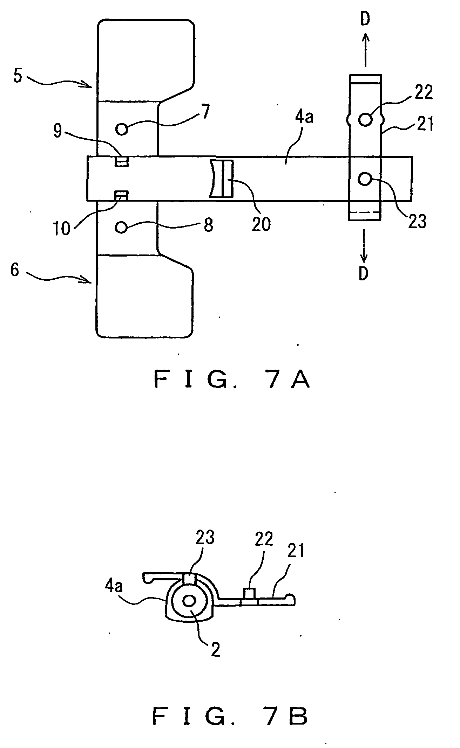

FIG. 7 shows a winged shield constituting a medical needle device according to a third embodiment. This winged shield includes an auxiliary holding mechanism including a pivot piece 21 at the base end portion of the shield cylinder 4a in addition to the configuration according to the embodiment 1. FIG. 7A is a plan view thereof and FIG. 7B is a sectional view taken on line D-D of FIG. 7A. The pivot piece 21 is fixed to the shield cylinder 4a at one side in the longitudinal direction thereof. Another side can pivot around the axis of the shield cylinder 4a and has a projection 22. On the fixed portion, a through hole 23 is formed. On the cylindrical wall of the shield tube 4a, through hole communicating to the through hole 23 is provided.

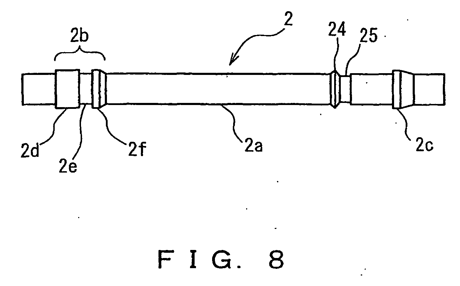

FIG. 8 shows a needle base 2 used in combination with the shield cylinder 4a shown in FIG. 7. On the rear portion of the needle base 2, an auxiliary holding portion including an annular projection 24 and an annular groove 25 is provided. In the state in which this needle base 2 is inserted into the winged shield of FIG. 7, and the stopper portion 2c is pressed into contact with the base end of the shield cylinder 4a, that is, in the state for use mentioned above, the position of the annular groove 25 of the auxiliary holding portion coincides with the position of the through hole 23 of the pivot piece 21.

In this state, by allowing the pivot piece 21 to pivot and allowing the projection 22 to be inserted into the through hole 23, the front end of the projection 22 is engaged with the annular groove 25. Thus, it is possible to provide a holding force for holding the needle base 2 with respect to the shield cylinder 4a. This holding force is provided in addition to a holding force obtained by the engagement between the front end projection 15, 16 of the shield cylinder 4a and the annular groove 2e of the needle base 2. In the case where the auxiliary holding force is not necessary, or a needle base 2 is stored in the shield cylinder 4a, a projection 22 of the pivot piece 21 is pulled from the through hole 23.

If the diameters of the projection 22 and the through hole 23 are properly set so that the projection 22 is pressed into the through hole 23, the state also can be fixed. Furthermore, the same holding effect can be obtained with only any one of the annular projection 24 and the annular groove 25.

With the above-mentioned embodiment, by strengthening the relatively weak holding force after sticking operation and by differentiating the holding force between at the time the needle is retained and at the time the needle is stored, it is possible to set two levels of holding force.

### (Fourth Embodiment)

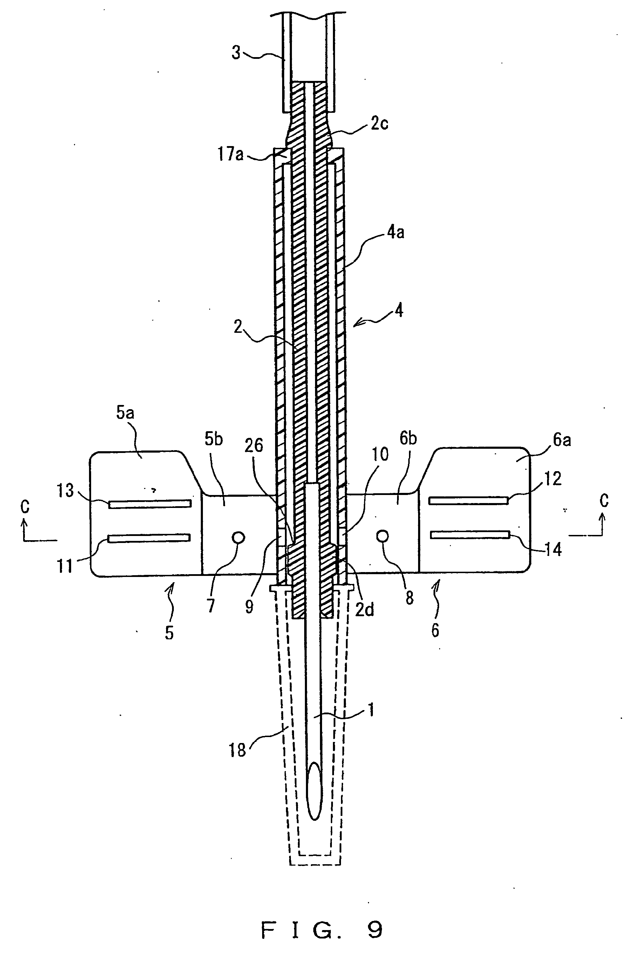

FIG. 9 shows a medical needle device according to the fourth embodiment. In this device, the holding portion of the needle base 2 is formed of only a large diameter portion 2d. At the rear end of the shield cylinder 4a, only a small diameter portion 17a is formed. The other configurations are the same as in the first embodiment.

In this configuration, with a step portion 26 formed at a boundary between the large diameter portion 2d of the needle base 2 and the rear portion thereof, the holding effect in use can be obtained. That is, the wing projections 7, 8 or the front end projections (not shown in FIG. 9) of the shield cylinder 4a are engaged with the step portion 26, thereby preventing the needle base 2 from moving toward the rear end side of the shield cylinder 4a. Thus, similar to the first embodiment, the holding of the needle base 2 at the time of the sticking operation can be carried out by the wing projections 7, 8 and the holding of the needle base 2 after sticking can be carried out by the front end projection of the shield cylinder 4a.

FIG. 9 does not show a structure for holding the needle base 2 in a state in which it is stored. However, such a holding effect may be obtained by using any one of the known structures.

### (Fifth Embodiment)

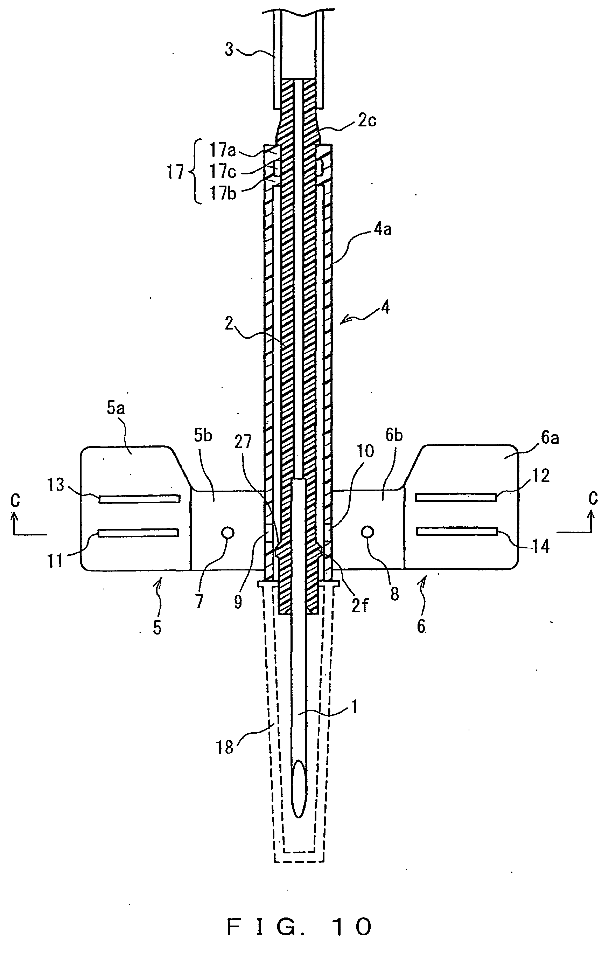

FIG. 10 shows a medical needle device according to a fifth embodiment. In this device, the holding portion of the needle base 2 is formed of only the outward annular projection 2f. The outward annular projection 2f is disposed such that it replaces the large diameter portion 2d of FIG. 1. Other configurations are the same as those in the first embodiment.

In this configuration, the holding effect can be obtained by the step portion 27 formed at the boundary between the needle base 2 and the outward annular projection 2f. That is, the wing projections 7, 8 or the front end projection (not shown in FIG. 10) of the shield cylinder 4a are engaged with the step portion 27, thereby preventing the needle base 2 from moving to the rear end side of the shield cylinder 4a. Thus, similar to the first embodiment, the holding of the needle base 2 at the time of the sticking operation can be carried out by the wing projections 7, 8, and the holding of the needle base 2 after sticking can be carried out by the front end projection of the shield cylinder 4a.

Furthermore, in a state in which the needle base 2 is stored, the outward annular projection 2f is engaged with the annular groove 17c formed on the rear end latch portion 17 of the shield cylinder 4a. Thus, the state in which the needle base 2 is stored is maintained.

### Industrial Applicability

According to the present invention, the holding of the needle base at the time of sticking operation is strong and reliable, thus enhancing the safety in sticking. Furthermore, at the time of sticking, only by carrying out the same operation as the operation of a general winged needle, it is possible to increase the force for holding the needle base without any particular operation. Therefore, the device can be handled easily.

## Claims

1. A medical needle device having a winged shield for preventing accidental needlesticks, comprising a winged shield having a substantially cylindrical shaped shield cylinder and a pair of wings connected to the front end side of the shield cylinder, a needle base inserted into an inner cavity of the shield cylinder movably in the axial direction, and a needle attached to the front end of the needle base, the needle being capable of being stored in the inner cavity of the shield cylinder with the front end thereof being covered, wherein
each of the wings has a wing projection protruding from the surface of the base region, and the shield cylinder has a through hole on the wall of the cylinder so that each of the wing projections can be inserted therein, and
by overlaying the wings along the side surface of the shield cylinder, each of the wing projections can be inserted into the inner cavity of the shield cylinder via the through hole, whereby the wing projections prevent the needle base from moving in the axis direction inside the shield cylinder, and the needle base is held in the shield cylinder in a state in which the needle protrudes from the front end of the shield cylinder by a predetermined length.

2. The medical needle device according to claim 1, wherein each of the wing projections is inserted into the inner cavity of the shield cylinder via the through hole to be brought into contact with the needle base, and with the friction force caused by the contact, the needle base is held in the shield cylinder in a state in which the needle protrudes from the front end of the shield cylinder by a predetermined length.

3. The medical needle device according to claim 1, wherein the pair of through holes corresponding respectively to the pair of wing projections are formed separately on both the walls of the shield cylinder.

4. The medical needle device according to claim 1, wherein the through hole is one groove formed continuously on both side surfaces of the shield cylinder.

5. The medical needle device according to claim 1, wherein in each of the wings the thickness of the base region is smaller than the thickness of the edge side region in each of the wings.

6. The medical needle device according to claim 1, wherein the outer surface of the bottom portion of the shield cylinder is formed flat.

7. The medical needle device according to claim 1, wherein the needle base comprises a main tube portion and a holding portion located in the vicinity of the front end of the main tube portion, the holding portion has a large diameter portion whose diameter is larger than the diameter of the main tube portion, and a step portion is formed on the outer surface of the rear portion of the large diameter portion;
by overlaying both the wings along the side surface of the shield cylinder, each of the wing projections can be engaged with the step portion of the needle base via the through hole, and
by engaging each of the wing projections with the step portion of the needle base, the needle base is held so that it does not move toward the base end side in the shield cylinder in a state in which the needle protrudes from the front end of the shield cylinder by a predetermined length.

8. The medical needle device according to claim 7, wherein the shield cylinder further comprises a rear end latch portion formed on the inner surface of the rear end, and the rear end latch portion has a smaller inner diameter than that of the large diameter part in the holding portion of the needle base, and an annular groove is formed in the middle portion in the axial direction, and
by moving the needle base in the axial direction toward the base end side with respect to the shield cylinder, the large diameter portion in the holding portion of the needle base can be engaged with the annular groove in the rear end latch portion of the shield cylinder, and the engagement enables the needle base to be held in the shield cylinder in a state in which the needle is stored in the inner cavity of the shield cylinder.

9. The medical needle device according to claim 7, wherein the holding portion of the needle base further comprises an outward annular projection at the rear portion of the large diameter portion so as to form an annular groove on the outer surface between the large diameter portion and the outward annular projection, and
each of the wing projections is inserted into the inner cavity of the shield cylinder via the through hole and can be engaged with the annular groove of the needle base, and the engagement enables the needle base to be held in the shield cylinder in a state in which the needle protrudes from the front end of the shield cylinder by a predetermined length.

10. The medical needle device according to claim 9, wherein the shield cylinder further comprises a rear end latch portion formed on the inner surface of the rear end, and the rear end latch portion comprises an inward annular projection; the inward annular projection can be engaged with the annular groove of the needle base by moving the needle base into the direction of the base end side with respect to the shield cylinder; and the engagement enables the needle base to be held in the shield cylinder in a state in which the needle is stored in the inner cavity of the shield cylinder.

11. The medical needle device according to claim 7, wherein the shield cylinder further comprises a front end projection formed on the inner surface at a position in the axial direction substantially corresponding to the through hole,
the front end projection of the shield cylinder can be engaged with the step portion of the needle base, and in the engagement state, by overlaying both the wings along the side surface of the shied cylinder, each of the projections can be engaged with the step portion of the needle base via the through hole, and
by engaging the front end projection or each of the wing projections with the step portion of the needle base, the needle base is held so that it is prevented from moving in the shield cylinder toward the base end side in a state in which the needle protrudes from the front end of the shield cylinder by a predetermined length.

12. The medical needle device according to claim 11, wherein the holding portion of the needle base further comprises an outward annular projection at the rear portion of the large diameter portion so as to form the annular groove on the outer surface between the large diameter portion and the outward annular projection, and
by engaging the front end projection of the shield cylinder or each of the wing projections of the annular groove of the needle base, the same function as the holding function based on the step portion can be obtained.

13. The medical needle device according to claim 11, wherein by pinching and pressing the wings at the position of the each of the projections in a state in which both the wings are overlaid along the side surface of the shield cylinder and the step portion of the needle base is engaged with each of the wing projections, the holding force with respect to the needle base is capable of being made larger than the holding force obtained by the engagement between the step portion of the needle base and the front end projection.

14. The medical needle device according to claim 1, wherein a holding piece is formed on the upper surface of the shield cylinder.

15. The medical needle device according to claim 1, further comprising an auxiliary holding mechanism provided on the base end of the shield cylinder and an auxiliary holding portion formed on the rear portion of the needle base, wherein the auxiliary holding mechanism comprises a pivot piece attached to the outer surface of the base end portion of the shield cylinder, an auxiliary projection provided on the pivot piece, and a through hole provided on the wall of the shield cylinder so that the auxiliary projection can be inserted into the inner cavity of the shield cylinder by way of pivoting of the pivot piece, and the auxiliary holding portion comprises an auxiliary annular groove or an auxiliary annular projection provided on the outer surface of the needle base,
by engaging the auxiliary projection with the auxiliary annular groove or auxiliary annular projection of the needle base via the through hole, the auxiliary holding force for holding the needle base in the shield cylinder is provided in a state in which the needle protrudes from the front end of the shield cylinder by a predetermined length.
